(19) Europäisches Patentamt
European Patent Office
Office européen des brevets

(11) **EP 3 645 771 B1**

(12) **EUROPÄISCHE PATENTSCHRIFT**

(45) Veröffentlichungstag und Bekanntmachung des Hinweises auf die Patenterteilung:
**08.01.2025 Patentblatt 2025/02**

(21) Anmeldenummer: **18733586.4**

(22) Anmeldetag: **21.06.2018**

(51) Internationale Patentklassifikation (IPC):
**D01D 5/06** (2006.01)          **D01F 8/02** (2006.01)
**D01D 1/02** (2006.01)

(52) Gemeinsame Patentklassifikation (CPC):
(C-Sets verfügbar)
**D01F 8/02; A61L 15/225; A61L 15/60; D01D 1/02; D01D 5/06** (Forts.)

(86) Internationale Anmeldenummer:
**PCT/EP2018/066610**

(87) Internationale Veröffentlichungsnummer:
**WO 2019/002095 (03.01.2019 Gazette 2019/01)**

(54) **VERFAHREN ZUR HERSTELLUNG EINER HYDROGEL-BILDENDEN MEHRKOMPONENTENFASER**

PROCESS FOR THE PREPARATION OF A HYDROGEL-FORMING MULTICOMPONENT FIBER

PROCÉDÉ DE PRÉPARATION DE FIBRE MULTICOMPOSANT FORMANT UN HYDROGEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.06.2017 DE 102017006025**

(43) Veröffentlichungstag der Anmeldung:
**06.05.2020 Patentblatt 2020/19**

(73) Patentinhaber: **Carl Freudenberg KG**
**69469 Weinheim (DE)**

(72) Erfinder:
- **KOSAN, Birgit**
  **07407 Rudolstadt (DE)**
- **MOOZ, Michael**
  **07318 Saalfeld (DE)**
- **MEISTER, Frank**
  **07407 Rudolstadt (DE)**

(56) Entgegenhaltungen:
CN-A- 103 060 946     US-A1- 2005 101 900
US-A1- 2016 121 013

(52) Gemeinsame Patentklassifikation (CPC): (Forts.)

C-Sets
**A61L 15/225, C08L 1/00;**
**A61L 15/225, C08L 1/26;**
**A61L 15/60, C08L 1/26**

## EP 3 645 771 B1

**Beschreibung**

Die Erfindung betrifft ein Verfahren zur Herstellung einer Hydrogel-bildenden Mehrkom ponentenfaser..

Hydrogel-bildende Fasern sind grundsätzlich bekannt. So beschreibt die GB 2518199 einen Prozess zur Herstellung quellbarer Fasern mit mindestens einem inkorporierten, antimikrobiellen Wirkstoff für die Wundversorgung. Hierbei wird ein quellbares, wasserlösliches Polymer unter Einbindung von Glykolen (mit 8 bis 15 C-Atomen), Glycerol, etc. mittels Nassextrusion durch Düsen zu quellbaren Fasern verformt. Als antimikrobielle Wirkstoffe werden Metall(salz) e über das Koagulationsbad in die Fasern eingebunden. Nachteilig an dem beschriebenen Verfahren ist, dass durch die Vernetzung mit den im Spinnbad enthaltenen Metallsalzen relativ spröde Fasern generiert werden, die nur mittels ausreichender Mengen an Weichmacher (Glykol, Glycerol, etc.) zu textilen Flächen (Gewebe, Gewirke, Vliese, etc.) verarbeitet werden können. Die Vernetzung ist notwendig um eine ausreichende Stabilität zu generieren. Ferner ist nachteilig, dass sich die Polymere bei der Auflösung chemisch verändern, sowie dass Weichmacher erforderlich sind.

Die US 2008/0082065 A beschreibt eine Kompositfaser aus Carboxymethylcellulose (CMC) (60 bis 99 %), einem Polygalaktomannan oder Polyglucomannan (1 bis 20 %) sowie Cellulose(kurz)fasern (im einfachsten Fall: Zellstofffasern - 2 bis 15 %), die durch Metall-ionen vernetzt werden. Ebenfalls beschrieben wird ein Verfahren zur Herstellung dieser Kompositfaser, bei dem die CMC und das Galakto- bzw. Glucomannan gemeinsam mit den Zellstofffasern in Wasser dispergiert und anschließend mit ausgewählten Metallionen vernetzt, das entstehende Gel anschließend in einem mit Wasser mischbaren Lösungsmittel aufgelöst und zu Fasern verformt wird und diese anschließend mit einem zweiten vernetzenden Metallsalz nochmals vernetzt werden, wobei spezielle, wässrige Metallsalzlösungen auf Basis von Al-, Ti-, Bi-, B- oder Zr-Verbindungen eingesetzt werden. Die Fasern werden durch Rühren der im Wasser mischbaren Lösungsmittel erzeugt. Nachteilig an dem beschriebenen Verfahren ist der Erhalt von wenig orientierten und wenig festen Fasern mit eingeschränktem Gelbildungsvermögen. Auch hier ist eine Vernetzung notwendig um eine ausreichende Stabilität zu generieren. Ferner ist nachteilig, dass sich die Polymere bei der Auflösung chemisch verändern, sowie dass Metallionen erforderlich sind.

Die US 2014/0322512 A1 beschreibt Kern-Mantel-Fasern aus einem hydrophoberen, festigkeitsgebenden Kern- und einem hydrophileren Mantelpolymeren, ein Verfahren zu ihrer Herstellung mittels Elektrospinnen sowie ihre Verwendung in medizinischen Artikeln. Das Mantelvolumen kann zwischen 100:1 bis 1:1 variieren. Das hydrophilere Mantelpolymer ist vernetzt. Hauptnachteil ist die eingeschränkte Haptik der oberflächlich vernetzten Fasern, die eingeschränkte Gelbildung der Faseroberfläche und die vergleichsweise geringe mechanische Festigkeit der Fasern. Nachteilig an dem Verfahren ist ferner, dass Elektrospinnen ein sehr teurer und bisher wenig effizienter Formgebungsprozess ist. Zudem ist die Fasergeometrie beschränkt auf Kern-Mantelfasern.

Die US 2005/0136253 A1 beschreibt einen Verformungsprozess für Hydroxylgruppen haltige(s) Polymer(e) (5 bis 100 % Stärke, Stärkederivate, PVA, etc.) in ausgewählten dipolar protischen und aprotischen Lösungsmitteln bzw. deren Mischungen mittels einer Zentrifugaldüse/Rotationsspinnen auch bei erhöhten Temperaturen. Ebenfalls beschrieben sind BiKo-Faserstrukturen (Kern-Mantel, Seite-an-Seite und diskontinuierliche statistische Mischungen beider Komponenten). Nachteilig an diesem Verfahren sind die eingeschränkten mechanischen Stabilitäten sowie das geringe Bindevermögen für Wasser oder wässrige Lösungen, insbesondere für 0,9 prozentige, wässrige Natriumchlorid-Lösung. Nachteilig ist ferner, dass die Festigkeit der erhaltenen Fasern relativ gering ist. Zudem ist die Materialauswahl beschränkt auf wasserlösliche Polymere, wobei anschließende Vernetzung erforderlich ist.

Bekannt sind auch carboxymethylierte Fasern, d.h. Fasern, die durch die nachträgliche Carboxymethylierung von Viskosefasern hergestellt werden (kommerziell erhältlich als "Aquacel-Fasern der Fa. Convatec; US 6 548730 B; AU 757461 B; WO 00/01425 A1; EP 1 091 770 A1). Diese Fasern ermöglichen zwar eine sehr hohe Wasseraufnahme, allerdings bilden sie bei Kontakt mit Wasser unter vollständigem Verlust der Faserstruktur ein Gel, was nicht für alle möglichen Anwendungen wünschenswert ist.

Ferner sind Viskosefasern mit einem Anteil an Carboxymethylcellulose (CMC) bekannt. Es handelt sich dabei um Mischfasern, die durch das Einspinnen von Carboxymethylcellulose in die Viskosespinnmasse erhalten werden. Solche Fasern wurden auch kommerziell hergestellt (US 4,199,367 A, US 4,289,824 A). Nachteilig an diesen Mischfasern ist eine mangelnde Verspinnbarkeit beziehungsweise eingeschränkte Faserfestigkeit bei hohen Anteilen an saugfähigem Polymer. Zudem ist das Wasseraufnahmevermögen beschränkt.

US 2005/101900 A1 beschreibt Polysaccharidfasern mit Wasserabsorptionseigenschaften, die durch den Einbau mindestens einer Substanz mit antimikrobiellen Eigenschaften in die Fasern gekennzeichnet sind und aus den Fasern gebildete Wundauflagen. Die Polysaccharidfasern werden vorzugsweise aus Alginat gebildet oder aus Alginat, das zusätzliche Polysaccharidmaterialien enthält, um ein zusätzliches Absorptionsvermögen zu ergeben (wie beispielsweise Natriumcarboxymethylcellulose). Alginate sind wasserlöslich. Mithin sind die Polysaccharidfasern aus zwei wasserlöslichen Fasermaterialien gebildet.

CN 103 060 946 A beschreibt Mischfasern aus Alginat und Natriumcarboxymethylcellulose. Auch diese Polysaccharidfasern sind aus zwei wasserlöslichen Fasermaterialien gebildet.

US 2016/121013 A1 beschreibt Mehrkomponentenfasern umfassend Pektin und ein proteinhaltiges Opfermaterial in einem Gewichtsverhältnis von Pektin zu proteinhaltigem Opfermaterial von 80:20 zu 60:40 und von 0 bis 90 Gew .% der Fasern eines anderen Polysaccharids oder eines wasserlöslichen Polymers. Als anderes Polysaccharid können Alginat, Chitosan oder seine Derivate oder Derivate von Cellulose, Guargummi, Xanthangummi, Johannisbrotkernmehl, Dextrin, Agar-Agar, Cellulosegummi oder ein anderes Material auf Stärkebasis eingesetzt werden. Die vorgenannten Polysaccharide/Polysaccharidderivate sind sämtlich wasserlöslich.

Der Erfindung liegt die Aufgabe zu Grunde ein Verfahren zur Herstellung einer Hydrogel-bildenden Faser bereitzustellen, mit der die oben beschriebenen Nachteile zumindest teilweise ausgeräumt werden können. Insbesondere soll die Faser in Kontakt mit verschiedenen Flüssigkeiten ein möglichst homogenes transparentes Gel bilden und dennoch ihre mechanische Integrität behalten. Diese Aufgabe wird gelöst durch ein Verfahren zur Herstellung einer Hydrogel-bildenden Mehrkomponentenfaser umfassend mindestens eine erste Faserkomponente und mindestens eine zweite Faserkomponente, wobei die erste und die zweite Faserkomponente unabhängig voneinander ausgewählt sind aus der Gruppe der Polysaccharide und/oder Polysaccharidderivate und wobei die erste Faserkomponente hergestellt wird aus einem Polysaccharid und/oder Polysaccharidderivat mit einer Wasserlöslichkeit, wie im Abschnitt Messmethoden definiert, von mehr als 50 Gew.%, vorzugsweise von mehr als 80 Gew.% und ganz besonders bevorzugt von mehr als 95 Gew.% und wobei die Mehrkomponentenfaser eine Wasserlöslichkeit, wie im Abschnitt Messmethoden definiert, von weniger als 15 Gew.% aufweist und wobei die zweite Faserkomponente hergestellt wird aus einem Polysaccharid und/oder Polysaccharidderivat mit einer Wasserlöslichkeit, wie im Abschnitt Messmethoden definiert, von 0 Gew.% bis 5 Gew.%.

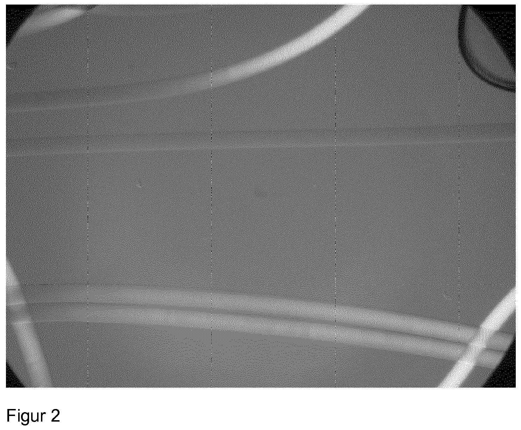

Die erfindungsgemäße Mehrkomponentenfaser ist eine Hydrogel-bildende Mehrkomponentenfaser. Der Begriff "Hydrogel" ist dabei im herkömmlichen Sinne zu verstehen als ein Wasser enthaltendes aber wasserunlösliches Polymer, dessen Moleküle chemisch, z. B. durch kovalente oder ionische Bindungen, oder Wasserstoffbrückenbindungen oder physikalisch, z. B. durch Verschlaufen der Polymerketten, zu einem dreidimensionalen Netzwerk verknüpft sind. Durch eingebaute hydrophile Polymerkomponenten quellen Hydrogel-bildende Fasern in Wasser und/oder wässrigen Lösungen unter beträchtlicher Volumenzunahme, ohne aber ihren stofflichen Zusammenhalt zu verlieren. Unter einer Hydrogel-bildenden Mehrkomponentenfaser ist mithin erfindungsgemäß eine Faser zu verstehen, die zumindest mit Wasser und/oder wässrigen Lösungen, insbesondere 0,9 prozentiger wässriger Natriumchlorid-Lösung und/oder Prüflösung A (nach DIN EN 13726-01, Abschnitt 3.2.2.3) ein Hydrogel bildet. Dabei quillt die Faser in diesen Lösungsmitteln unter Volumenzunahme ohne aber ihren stofflichen Zusammenhalt zu verlieren, so dass sie immer noch eine optisch erkennbare Faserform aufweist. In praktischen Versuchen wurde gefunden, dass die erfindungsgemäße Mehrkomponentenfaser in Prüflösung A ein homogenes transparentes "Faser-Gel" bildet, wie beispielsweise in Figur 2 demonstriert. Insofern kann die erfindungsgemäße Mehrkomponentenfaser auch als "Faser-Gel" bildende Mehrkomponentenfaser angesehen werden.

Ein Merkmal der erfindungsgemäßen Mehrkomponentenfaser ist mithin, dass sie zumindest in Wasser und/oder wässrigen Lösungen, insbesondere 0,9 prozentiger wässriger Natriumchlorid-Lösung und/oder Prüflösung A, unter Volumenzunahme quillt, beispielsweise unter Vergrößerung des Faserdurchmessers (20°C, Prüflösung A, nach 10 Minuten) um mehr als 100%, beispielsweise von 100% bis 600% und/oder von 150% bis 500% und/oder von 200% bis 400%.

Ein weiteres Merkmal der erfindungsgemäßen Mehrkomponentenfaser ist, dass sie beim Quellen in Wasser und/oder wässrigen Lösungen, insbesondere 0,9 prozentiger wässriger Natriumchlorid-Lösung und/oder Prüflösung A ihren stofflichen Zusammenhalt nicht verliert, so dass sie auch nach dem Quellen immer noch eine optisch erkennbare Faserform aufweist ("Faser-Gel").

Ein weiteres Merkmal der erfindungsgemäßen Mehrkomponentenfaser ist, dass sie eine Wasserlöslichkeit von weniger als 15 Gew.%, beispielsweise von 0 Gew.% bis 15 Gew.%, bevorzugt von 0 Gew.% bis 10 Gew.%, noch bevorzugter von 0 Gew.% bis 5 Gew.% und insbesondere von 0 Gew.% bis 3 Gew.% aufweist. Dies ist überraschend, da die erste Faserkomponente ausgehend von einem wasserlöslichen Polysaccharid und/oder Polysaccharidderivat hergestellt wird - umso mehr, da gefunden wurde, dass für die Einstellung der geringen Wasserlöslichkeit keine chemische Vernetzung der Mehrkomponentenfaser über kovalente und/oder ionische Bindungen nötig ist und vorzugsweise nicht durchgeführt wird. Vorteilhaft an einem Verzicht auf chemische Vernetzung ist, dass beim Einsatz der Fasern, beispielsweise in der Wundversorgung, keine durch die Vernetzung eingebrachten Fremdsubstanzen freigesetzt werden können.

Ohne sich erfindungsgemäß auf einen Mechanismus festzulegen wird vermutet, dass die Verringerung der Wasserlöslichkeit der Mehrkomponentenfaser gegenüber der als Ausgangsmaterial verwendeten ersten Faserkomponente darauf zurückzuführen ist, dass die erste und die zweite Faserkomponente eine Mischphase ausbilden und hierdurch einander stabilisieren. Besonders gut kann eine solche Stabilisierung erfolgen, wenn die Mischphase so ausgebildet ist, dass die erste und die zweite Faserkomponente miteinander Verschlingungs- und/oder Verschlaufungsnetzwerke bilden. Unter einem Verschlingungs- und/oder Verschlaufungsnetzwerk ist dabei ein physikalisches Netzwerk zu verstehen, das durch gegenseitiges Durchdringen von Makromolekülen der Polysaccharid und Polysaccharidderivate an der Phasengrenze zwischen der ersten und der zweiten Faserkomponente gebildet wird, sodass die Faserkomponenten keine klaren Phasengrenzen ausbilden. Besonders bevorzugt liegen dabei die Faserkomponenten statistisch verteilt vor.

Erhältlich ist eine solche Mischphase bzw. ein solches Verschlingungs- oder Verschlaufungsnetzwerk beispielsweise dadurch, dass die Hydrogel-bildende Mehrkomponentenfaser aus einer Spinnlösung hergestellt wird, die durch gemeinsame Auflösung mindestens einer ersten und einer zweiten Polymerkomponente erhalten wird, wobei die erste und die zweite Polymerkomponente unabhängig voneinander ausgewählt sind aus der Gruppe der Polysaccharide und/oder Polysaccharidderivate und wobei die erste Polymerkomponente ein Polysaccharid und/oder Polysaccharidderivat mit einer Wasserlöslichkeit von mehr als 50 Gew.% ist.

Die erfindungsgemäße Hydrogel-bildende Mehrkomponentenfaser zeichnet sich durch hervorragende mechanische Eigenschaften, insbesondere Reißfestigkeit, Reißdehnung und ein gutes Aufnahmevermögen für Prüflösung A aus. So weist die erfindungsgemäße Mehrkomponentenfaser in einer bevorzugten Ausführungsform der Erfindung eine feinheitsbezogene Reißfestigkeit im konditionierten Zustand zwischen 10 cN/tex und 40 cN/tex, noch bevorzugter zwischen 15 cN/tex und 30 cN/tex und/oder eine feinheitsbezogene Reißdehnung im konditionierten Zustand zwischen 5% und 15%, noch bevorzugter zwischen 5% und 10% und/oder ein Aufnahmevermögen für Prüflösung A zwischen 5 g/g und 30 g/g, noch bevorzugter zwischen 15 g/g und 30 g/g, auf.

Die erfindungsgemäße Mehrkomponentenfaser weist mindestens zwei Faserkomponenten auf. Diese sind vorzugsweise in ihrer chemischen Struktur unterschiedlich. Wie oben erläutert, liegen die beiden Faserkomponenten vorzugsweise als Mischphase vor, da derartige Fasern besonders kostengünstig und einfach, beispielsweise mit dem erfindungsgemäßen Verfahren, hergestellt werden können. Die Mischphase kann dabei in den verschiedensten Anordnungen in der Faser vorliegen. Beispielsweise kann sie als Komponente einer Kern-Mantel-Anordnung, einer segmentierten Anordnung, insbesondere einer Segmented Pie, Side-by-Side- und/oder Island-in-the-sea-Anordnung vorliegen. Ebenfalls bevorzugt liegt sie als Komponente einer Segmented pie- oder Island-in-the-sea-Anordnung vor, da bei diesen Anordnungen die Komponenten frei quellen können.

Im Falle einer Kern-Mantel-Anordnung, liegt die Mischphase bevorzugt im Mantel vor, da hierdurch eine Hydrogel-bildende Komponente der Faser auf der Außenseite vorliegt und den Charakter der Faser bestimmen kann. Bei einer Island-in-the-sea-Anordnung stellt die Hydrogel-bildende Komponente bevorzugt die "Sea" dar.

Erfindungsgemäß besonders bevorzugt bildet die Mischphase die Hauptkomponente der Faser. Mithin besteht die Faser vorzugsweise zu mehr als 90 Gew.%, noch bevorzugter von 95 Gew.%. bis 95 Gew.% aus der Mischphase.

In einer weiteren bevorzugten Ausführungsform der Erfindung weist die Hydrogel-bildende Mehrkomponentenfaser im trockenen Zustand einen Anteil an Polysacchariden und/oder Polysaccharidderivaten von mehr als 95 Gew.%, noch bevorzugter von mehr als 97 Gew.%, insbesondere von mehr als 99 Gew.%, jeweils bezogen auf das Gesamtgewicht der Mehrkomponentenfaser, auf.

Die erfindungsgemäße Mehrkomponentenfaser weist im trockenen Zustand vorzugsweise einen Faserdurchmesser zwischen 10 µm und 100 µm auf. In einer bevorzugten Ausführungsform der Erfindung liegt der Faserdurchmesser im Bereich zwischen 10 µm und 50 µm, noch bevorzugter zwischen 10 µm und 25 µm.

Das Mengenverhältnis von erster und zweiter Faserkomponente liegt vorzugsweise zwischen 80:20 und 10:90, noch bevorzugter zwischen 70:30 und 20:80 und ganz besonders bevorzugt zwischen 65:35 und 50:50.

Erfindungsgemäß ist die erste Faserkomponente ausgewählt aus der Gruppe der Polysaccharide und/oder Polysaccharidderivate, wobei die erste Faserkomponente hergestellt wird aus einem Polysaccharid und/oder Polysaccharidderivat mit einer Wasserlöslichkeit, wie im Abschnitt Messmethoden definiert, von mehr als 50 Gew.%. Erfindungsgemäß besonders bevorzugt ist die erste Faserkomponente ausgewählt aus der Gruppe der Cellulosederivate beispielsweise ionischer oder nicht-ionischer Cellulosesether, insbesondere Carboxymethylcellulose und/oder Hydroxypropylcellulose. Erfindungsgemäß besonders geeignete Polysaccharidderivate für die erste Faserkomponente sind Carboxyalkylcellulose, insbesondere Carboxymethylcellulose, Hydroxyalkylcellulose, insbesondere Hydroxypropylcellulose und/oder Sulfoalkylcellulose. Vorteilhaft an ihnen ist ihre gute Löslichkeit in protischen Lösungsmitteln, bevorzugt in Wasser.

Erfindungsgemäß ist die zweite Faserkomponente ebenfalls ausgewählt aus der Klasse der Polysaccharide und/oder Polysaccharidderivate, wobei Polysaccharide aufgrund ihrer verglichen mit Polysaccharidderivaten geringeren Löslichkeit bevorzugt sind. Erfindungsgemäß wird die zweite Faserkomponente hergestellt aus einem Polysaccharid und/oder Polysaccharidderivat mit einer Wasserlöslichkeit, wie im Abschnitt Messmethoden definiert, von 0 Gew.% bis 5 Gew.%, und besonders bevorzugt mit einer Wasserlöslichkeit von 0 Gew.% bis 1 Gew.%.

Die Polysaccharide der zweiten Faserkomponente können ionische oder bevorzugt nichtionische Polysaccharide sein, besonders bevorzugt Cellulose.

Grundsätzlich kann die Mehrkomponentenfaser die verschiedensten Kombinationen aus erster und zweiter Faserkomponente aufweisen. Von Vorteil ist es dabei, wenn die beiden Faserkomponenten wie unten dargelegt gemeinsam in einem Lösungsmittel aufgelöst werden können. Bevorzugt werden Kombinationen von ionischen oder nicht-ionischen Cellulosesethern, insbesondere Carboxymethylcellulose und/oder Hydroxypropylcellulose als erster Faserkomponente mit Cellulose als zweite Faserkomponente.

In einer weiteren bevorzugten Ausführungsform der Erfindung sind die Mehrkomponentenfasern Stapelfasern, die vorzugsweise eine Faserlänge im trockenen Zustand von 1 mm bis 60 mm, noch bevorzugter von 3 mm bis 40 mm, aufweisen. Diese sind für die Verarbeitung zu Stapelfaservliesstoffen besonders geeignet.

Die Hydrogel-bildende Mehrkomponentenfaser kann mithilfe der üblichen Verfahren zu textilen Flächengebilden verarbeitet werden. Ein weiterer Gegenstand der vorliegenden Erfindung ist mithin ein textiles Flächengebilde, beispielsweise ein Gewebe, Gewirke, Gestrick und/oder ein Vliesstoff, insbesondere ein Stapelfaservliesstoff, das die erfindungsgemäße Hydrogel-bildende Mehrkomponentenfaser aufweist und vorzugsweise zu mehr als 80 Gew.%, noch bevorzugter zu mehr als 90 Gew.%, und insbesondere zu mehr als 95 Gew.% aus erfindungsgemäßen Hydrogelbildenden Mehrkomponentenfasern besteht.

Die erfindungsgemäße Mehrkomponentenfaser eignet sich hervorragend zur Herstellung von Materialien für medizinische Anwendungen, insbesondere zur Herstellung von Wundauflagen, Wundverbänden, Nahtmaterialien, Implantaten, Tissue Engineering Scaffolds, Arzneimitteln, Trägermaterialien, Hygieneprodukten, insbesondere Damenhygieneprodukten, Windeln und Inkontinenzprodukten und/oder Kosmetikprodukten.

Ein bevorzugtes Verfahren zur Herstellung der erfindungsgemäßen Hydrogel-bildenden Mehrkomponentenfaser mit einer Wasserlöslichkeit von weniger als 15 Gew.%, umfasst folgende Schritte:

- Herstellung mindestens einer Spinnlösung durch die gemeinsame Auflösung in einem Lösungsmittel mindestens einer ersten Polymerkomponente und mindestens einer zweiten Polymerkomponente, wobei die erste und die zweite Polymerkomponente unabhängig voneinander ausgewählt ist aus der Gruppe der Polysaccharide und/oder Polysaccharidderivate und wobei die erste Polymerkomponente eine Wasserlöslichkeit von mehr als 50 Gew.%, vorzugsweise von mehr als 80 Gew.% und ganz besonders bevorzugt von mehr als 95 Gew.%, aufweist;
- Zuführen der Spinnlösung zu einer Spinndüse und Extrusion der Spinnlösung durch die Spinndüse in einen Luftspalt zur Erzeugung einer Mehrkomponentenstruktur;
- Einführen der Mehrkomponentenstruktur in ein Fällbad zur Ausbildung der Mehrkomponentenfaser;
- Entfernung des Lösungsmittels in mindestens einem Waschbad und
- Trocknung der Mehrkomponentenfaser.

Vorzugsweise kann mit dem erfindungsgemäßen Verfahren eine wie hier beschriebene erfindungsgemäße Mehrkomponentenfaser hergestellt werden.

Wesentliches Merkmal des erfindungsgemäßen Verfahrens ist, dass in einem ersten Verfahrensschritt eine Spinnlösung hergestellt wird durch gemeinsame Auflösung in einem Lösungsmittel mindestens einer ersten und mindestens einer zweiten Polymerkomponente, wobei die erste und die zweite Polymerkomponenten unabhängig voneinander ausgewählt sind aus der Gruppe der Polysaccharide und/oder Polysaccharidderivate und wobei die erste Polymerkomponente eine Wasserlöslichkeit von mehr als 50 Gew.% aufweist.

Ohne sich erfindungsgemäß auf einen Mechanismus festzulegen wird vermutet, dass die gemeinsame Auflösung der ersten und der zweiten Faserkomponente zu einer wechselseitigen Durchdringung und zur Ausbildung einer Mischphase bzw. von Verschlingungs- oder Verschlaufungsnetzwerken zwischen den Faserkomponenten in der erfindungsgemäßen Mehrkomponentenfaser führt. Hierdurch kann überraschenderweise eine signifikante Verringerung der Wasserlöslichkeit der Mehrkomponentenfaser gegenüber den als Ausgangsmaterialien verwendeten wasserlöslichen Polysacchariden und/oder Polysaccharidderivaten erhalten werden, was zu den oben beschrieben Vorteilen führt.

Überraschend ist insbesondere, dass die geringe Wasserlöslichkeit der Mehrkomponentenfaser auch ohne chemische Änderung der Polymerkomponenten erzielt werden kann. So kann auf eine Vernetzung, insbesondere durch chemische Vernetzer, der ersten Polymerkomponente verzichtet werden. Darüber hinaus kann durch die gemeinsame Auflösung besonders gut eine Faser hergestellt werden, bei der die Faserkomponenten eine Anordnung in Mischphasen aufweisen.

Ferner ist es möglich die Mengenverhältnisse zwischen der ersten und der zweiten Polymerkomponente sehr genau einzustellen. Diese liegen vorzugsweise zwischen 80:20 und 10:90, noch bevorzugter zwischen 70:30 und 20:80 und ganz besonders bevorzugt zwischen 65:35 und 50:50.

Durch Variation der Polymeranteile und/oder gezielte Auswahl der Polymerkomponenten in der Spinnlösung können auf einfache Weise die Fasereigenschaften eingestellt werden, beispielsweise das Feuchteaufnahmevermögen und die textilphysikalischen Eigenschaften.

In einer bevorzugten Ausführungsform der Erfindung findet die Auflösung der ersten und der zweiten Polymerkomponente in Form einer Direktauflösung statt. Die Direktauflösung ist ein bekanntes Verfahren und beispielsweise in T. Röder, R. Möslinger, U. Mais, B. Morgenstern, U. Glatter" Charakterisierung der Lösungsstrukturen in technisch relevanten Celluloselösungen", Lenzinger Berichte 82 (2003) 118-127, in R. Maron, Ch. Michels, E. Taeger, "Investigations for preparation of cellulose solutions in NMMO and the following forming", Lenzinger Berichte 74 (1994), 27-29, oder in D. Cole, A. Jones, "Solvent-Spun Fibre - A New Member of the Cellulose Fibre Family", Lenzinger Berichte 69 (1990), 73-77 beschrieben.

Als Lösungsmittel können die verschiedensten Lösungsmittel eingesetzt werden, die bevorzugt eine Direktauflösung der ersten Polymerkomponente und/oder der zweiten Polymerkomponenten ermöglichen. Besonders geeignet zu diesem Zweck sind N- Methyl-Morpholin-N-Oxid Monohydrat, aber auch ionische Flüssigkeiten wie beispielsweise EMIMAc, BMIMCl.

In einem weiteren Verfahrensschritt finden das Zuführen der Spinnlösung zu einer Spinndüse und die Extrusion der Spinnlösung durch die Spinndüse in einen Luftspalt statt. Als Spinndüse kann eine Ein- oder Mehrkomponentenspinndüse eingesetzt werden. In dem Luftspalt findet eine Verstreckung der extrudierten Mehrkomponentenstruktur statt.

In einem weiteren Verfahrensschritt findet das Einführen der Mehrkomponentenstruktur in ein Fällbad zur Ausbildung der Mehrkomponentenfaser statt. Das Fällbad dient einer simultanen Regeneration/Koagulation der Mehrkomponentenstruktur unter Bildung der erfindungsgemäßen Mehrkomponentenfaser. Bevorzugt enthält das Fällbad wässrige Salzlösungen oder organisch-wässrige Lösungen des zur Auflösung der Polymerkomponenten verwendeten Lösungsmittels. Die wässrigen Salzlösungen weisen vorzugsweise Sulfate, insbesondere Natriumsulfat, Zinksulfat auf. Der Salzgehalt des Fällbads beträgt bevorzugt bis zu 20 Gew.%, noch bevorzugter 1 bis 10 Gew.%, und insbesondere 1 bis 5 Gew.%. Das Verhältnis zwischen dem zur Auflösung der Polymerkomponenten verwendeten Lösungsmittels und Wasser liegt vorzugsweise zwischen 15:85 und 35:65.

In einem weiteren Verfahrensschritt findet die Entfernung des Lösungsmittels in mindestens einem Waschbad statt. Das mindestens eine Waschbad enthält bevorzugt Wasser, das zur Auflösung der Polymerkomponenten verwendete Lösungsmittel, ein organisches Lösungsmittel wie Alkohol, Aceton und/oder Mischungen davon. In einer bevorzugten Ausführungsform werden mehrere Waschbäder verwendet, die einen unterschiedlichen Gehalt an organischem Lösungsmittel aufweisen. Durch die Verwendung von organischen Lösungsmitteln, die die erste und/oder zweite Polymerkomponente nicht lösen, kann die Mehrkomponentenfaser stabilisiert werden.

In einem weiteren Verfahrensschritt findet die Trocknung der Mehrkomponentenfaser statt.

Das erfindungsgemäße Verfahren zeichnet sich dadurch aus, dass es technisch-technologisch einfach durchzuführen und einfach höher skalierbar ist. Durch das erfindungsgemäße Verfahren können, wie oben erläutert, Hydrogel-bildende Mehrkomponentenfasern mit hoher mechanischer Integrität insbesondere im feuchten und/oder gequollenen Zustand erhalten werden.

Mit dem erfindungsgemäßen Verfahren kann ferner die erfindungsgemäße Mehrkomponentenfaser, beispielsweise in einer Anordnung in makroskopischen Mischphasen auf einfache Weise bewirkt werden, da sie bereits durch die Lösungsherstellung vorgebildet ist.

Gegebenenfalls können die Mehrkomponentenfasern auf die gewünschte Stapellänge (bevorzugt 1 bis 60 mm) geschnitten werden. Es können sich übliche Nachbehandlungsschritte wie Bleichen, Avivieren oder Kräuseln anschließen.

Mit dem hier beschriebenen Verfahren kann eine erfindungsgemäße Mehrkomponentenfaser hergestellt werden

Figurenbeschreibung

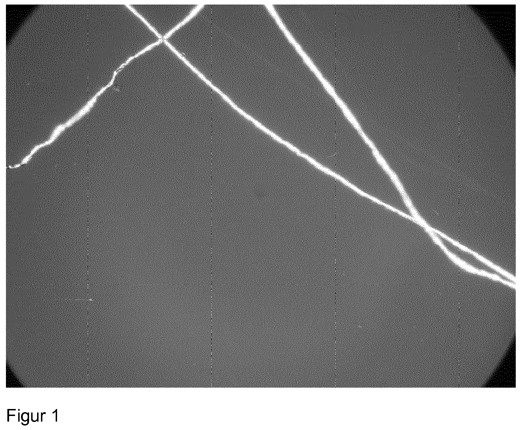

Figur 1 zeigt die fotografische Aufnahme dreier erfindungsgemäßer Mehrkomponentenfasern im getrockneten Zustand.

Figur 2 zeigt die fotografische Aufnahme mehrerer erfindungsgemäßer Mehrkomponentenfasern im gequollenen Zustand. Die Quellung wurde mittels Prüflösung A herbeigeführt. Es ist deutlich zu erkennen, dass die Fasern gequollenen sind ohne aber ihren stofflichen Zusammenhalt verloren zu haben, so dass sie immer noch eine optisch erkennbare Faserform aufweisen (Fasergel). In diesen Fasern sind die Faserkomponenten in einer Mischphase angeordnet.

Im Folgenden sind die zur Bestimmung der in dieser Erfindung verwendeten Messmethoden aufgeführt:
**Wasserlöslichkeit des Polysaccharids und/oder Polysaccharidderivats:** Die Wasserlöslichkeit wird mittels Hausvorschrift ermittelt. Etwa 2 g des Polysaccharids bzw. Polysaccharidderivats, dessen Trockengehalt (TG in %) in einer separaten Probe bei 105°C ermittelt wurde, werden analytisch genau eingewogen (Einwaage PS in g), mit 100 ml deionisiertem Wasser versetzt und die Probe für 30 min bei Raumtemperatur gerührt. Anschließend wird die Probe über eine G3-Fritte, welche vorher bei 105°C getrocknet und ausgewogen wurde (A in g), filtriert, der Filterkuchen mit Fritte bei 105°C bis zur Gewichtskonstanz getrocknet und ausgewogen (B in g). Die Wasserlöslichkeit des Polysaccharids bzw. Polysaccharidderivats ergibt sich zu:

$$Wasserlöslichkeit\ des\ Polsacchariad\,(derivat)s\ [\%] = \left(1 - \frac{B-A}{PS \times {}^{TG}/_{100}}\right) \times 100$$

**Wasserlöslichkeit der Mehrkomponentenfaser:**

Die Wasserlöslichkeit wird mittels Hausvorschrift ermittelt. Etwa 2 g der Mehrkomponentenfaser, deren Trockengehalt (TG in %) in einer separaten Probe bei 105°C ermittelt wurde, werden analytisch genau eingewogen (Einwaage F in g), mit 100 ml deionisiertem Wasser versetzt und die Probe für 30 min bei Raumtemperatur gerührt. Anschließend wird die Faser über eine G3-Fritte, welche vorher bei 105°C getrocknet und ausgewogen wurde (A in g), filtriert, die Faser mit Fritte bei 105°C bis zur Gewichtskonstanz getrocknet und ausgewogen (B in g). Die Wasserlöslichkeit der Mehrkomponentenfaser ergibt sich zu:

$$Wasserlöslichkeit\ der\ Mehrkomponentenfaser\ [\%] = \left(1 - \frac{B-A}{F \times {}^{TG}/_{100}}\right) \times 100$$

**Faserdurchmesser**

Der Faserdurchmesser der getrockneten bzw. gequollenen Mehrkomponentenfasern wurde durch Ausmessen mittels Mikroskop ermittelt.

**Feinheitsbezogene Reißfestigkeit und Reißdehnung im konditionierten Zustand:**

Die feinheitsbezogene Reißfestigkeit und Reißdehnung im konditionierten Zustand werden mittels DIN EN ISO 5079 bestimmt.

**Faserfeinheit:**

Die Faserfeinheit wird mittels DIN EN ISO 1973 bestimmt.

**Aufnahmevermögen für Blutersatzflüssigkeit (Prüflösung A):**

Die Bestimmung des Aufnahmevermögens für Blutersatzflüssigkeit erfolgt in Anlehnung an die DIN 53923. Statt der in der DIN 53923 beschriebenen Aufnahme an Wasser wird die Aufnahme an Prüflösung A bestimmt.

**Prüflösung A nach DIN EN 13726-01, Abschnitt 3.2.2.3**

Prüflösung A, enthaltend Natriumchlorid- und Calciumchloridlösung mit einem Gehalt von 142 mmol Natriumionen und 2,5 mmol Calciumionen als Chloride weist eine dem menschlichen Serum oder Wundsekret vergleichbare Ionenzusammensetzung auf. Sie wird durch Lösen von 8,298 g Natriumchlorid und 0,368 g Calciumchlorid-Dihydrat in deionisiertem Wasser und Auffüllen auf 1 l in einem Messkolben hergestellt.

Im Folgenden wird die Erfindung anhand eines Beispiels näher erläutert:

**Beispiel: Herstellung einer erfindungsgemäßen Mehrkomponentenfaser aus Natriumcarboxymethylcellulose als erster Polymerkomponente und aus Cellulose als zweiter Polymerkomponente im Verhältnis 50/50**

17,47 g Cellulose (Eukalyptus-Sulfit-Zellstoff, Cuoxam-DP: 500, Trockengehalt (TG): 93%) (zweite Polymerkomponente) werden mittels Ultra-Turrax in Wasser bis zur Einzelfaser aufgeschlagen und auf einen Trockengehalt von 35% abgepresst. 17,66 g Na-CMC (DS: 0,9, MW: 250.000, TG: 92%) (erste Polymerkomponente) werden mittels Ultra-Turrax in 377 g 50%iges N-Methylmorpholin-N-oxid (NMMO), enthaltend 0,16 g Propylgallat als Stabilisator, eingerührt und nach Zugabe des abgepressten Zellstoffes gemeinsam in eine homogene Suspension überführt. Diese Suspension wird in einen Laborkneter überführt und unter Scherung, Temperatur und Vakuum durch Entfernung des überschüssigen Wassers bis zum NMMO-Monohydrat in eine homogen verteilte Blendpolymerlösung mit einer Nullscherviskosität von 6000 Pas bei 85°C überführt. Mittels Trocken-Nass-Spinnprozess wird die Blendpolymerlösung zu Mehrkomponentenfasern einer Feinheit von 1,64 dtex mit den aufgeführten Fasereigenschaften unter Nutzung folgender Spinnbedingungen verformt:

| Spinnbedingungen: | | |
|---|---|---|
| Spinnmassetemperatur | °C | 79 |
| Düsenlochdurchmesser | μm | 100 |
| Kapillaranzahl | | 30 |
| Spinndruck | bar | 30 |
| Luftspalt | mm | 40 |
| Fällbad | | 5% $Na_2SO_4$ |
| Spinngeschwindigkeit | m/min | 30 |
| Waschbäder | | Wasser/Ethanol 75/25, 50/50, 25/75 |
| | | |
| Faserprüfung: | | |
| Feinheit | dtex | 1,77 |
| Feinheitsbezogene Reißfestigkeit, kond. | cN/tex | 24,5 |
| Reißdehnung, kond. | % | 8,2 |
| Aufnahmevermögen Prüflösung A | g/g | 21,4 |

Es zeigt sich, dass die erfindungsgemäße Hydrogel-bildende Mehrkomponentenfaser eine hervorragende feinheitsbezogene Reißfestigkeit und Reißdehnung sowie ein hervorragendes Aufnahmevermögen für Prüflösung A aufweist.

**Patentansprüche**

1. Verfahren zur Herstellung einer Hydrogel-bildenden Mehrkomponentenfaser umfassend mindestens eine erste Faserkomponente und mindestens eine zweite Faserkomponente, wobei die erste und die zweite Faserkomponente unabhängig voneinander ausgewählt sind aus der Gruppe der Polysaccharide und/oder Polysaccharidderivate und wobei die erste Faserkomponente hergestellt wird aus einem Polysaccharid und/oder Polysaccharidderivat mit einer Wasserlöslichkeit, wie im Abschnitt Messmethoden definiert, von mehr als 50 Gew.%, vorzugsweise von mehr als 80 Gew.% und ganz besonders bevorzugt von mehr als 95 Gew.% und wobei die Mehrkomponentenfaser eine Wasserlöslichkeit, wie im Abschnitt Messmethoden definiert, von weniger als 15 Gew.% aufweist, **dadurch gekennzeichnet, dass** die zweite Faserkomponente hergestellt wird aus einem Polysaccharid und/oder Polysaccharidderivat mit einer Wasserlöslichkeit, wie im Abschnitt Messmethoden definiert, von 0 Gew.% bis 5 Gew.%.

2. Verfahren nach Anspruch 1, **dadurch gekennzeichnet, dass** die Hydrogel-bildende Mehrkomponentenfaser keine chemische Vernetzung der ersten und/oder zweiten Faserkomponente über kovalente und/oder ionische Bindungen aufweist.

3. Verfahren nach Anspruch 1 oder 2, **dadurch gekennzeichnet, dass** die Hydrogel-bildende Mehrkomponentenfaser in Prüflösung A (nach DIN EN 13726-01, Abschnitt 3.2.2.3) unter Vergrößerung des Faserdurchmessers (20°C, Prüflösung A, nach 10 Minuten) um mehr als 100% quillt.

4. Verfahren nach einem oder mehreren der vorangehenden Ansprüche, **dadurch gekennzeichnet, dass** die erste und die zweite Faserkomponente als Mischphase vorliegen und vorzugsweise miteinander Verschlingungs- und/oder Verschlaufungsnetzwerke ausbilden.

5. Verfahren nach Anspruch 4, **dadurch gekennzeichnet, dass** die Mischphase und/oder das Verschlingungs- und/oder Verschlaufungsnetzwerk als Komponente einer Kern-Mantel-Anordnung, einer segmentierten Anordnung, insbesondere einer Segmented Pie, Side-by-Side- und/oder Island-in-the-sea-Anordnung vorliegen.

6. Verfahren nach einem oder mehreren der vorangehenden Ansprüche, **dadurch gekennzeichnet, dass** die Hydrogel-bildende Mehrkomponentenfaser durch gemeinsame Auflösung mindestens einer ersten und einer zweiten Polymerkomponente erhalten wird, wobei die erste und die zweite Polymerkomponente unabhängig voneinander ausgewählt sind aus der Gruppe der Polysaccharide und/oder Polysaccharidderivate und wobei die erste Polymerkomponente ein Polysaccharid und/oder Polysaccharidderivat mit einer Wasserlöslichkeit, wie im Abschnitt Messmethoden definiert, von mehr als 50 Gew.% ist.

7. Verfahren nach einem oder mehreren der vorangehenden Ansprüche, **dadurch gekennzeichnet, dass** die Hydrogel-bildende Mehrkomponentenfaser eine feinheitsbezogene Reißfestigkeit im konditionierten Zustand zwischen 10 cN/tex und 40 cN/tex und/oder eine feinheitsbezogene Reißdehnung im konditionierten Zustand zwischen 5% und 15% und/oder ein Aufnahmevermögen, gemessen in Anlehnung an die DIN 53923 für Prüflösung A (nach DIN EN 13726-01, Abschnitt 3.2.2.3) zwischen 5 g/g und 30 g/g aufweist.

8. Verfahren nach einem oder mehreren der vorangehenden Ansprüche, **dadurch gekennzeichnet, dass** das Mengenverhältnis von erster und zweiter Faserkomponente zwischen 80:20 und 10:90 liegt.

9. Verfahren nach einem oder mehreren der vorangehenden Ansprüche, **dadurch gekennzeichnet, dass** die erste Faserkomponente ausgewählt ist aus der Gruppe der Cellulosederivate beispielsweise Cellulosesether, insbesondere Carboxymethylcellulose, Hydroxypropylcellulose und/oder Sulfoalkylcellulose und/oder dass die zweite Faserkomponente ausgewählt ist aus der Gruppe der Polysaccharide und/oder Polysaccharidderivate mit einer Wasserlöslichkeit von 0 Gew.% bis 5 Gew.%..

10. Verfahren nach einem oder mehreren der vorangehenden Ansprüche, **dadurch gekennzeichnet, dass** die erste Faserkomponente ausgewählt ist aus der Gruppe der Cellulosesether, insbesondere Carboxymethylcellulose, Hydroxypropylcellulose und/oder Sulfoalkylcellulose und/oder dass die zweite Faserkomponente ausgewählt ist aus der Gruppe der nichtionischen Polysaccharide, insbesondere Cellulose.

11. Verfahren nach einem oder mehreren der vorangehenden Ansprüche , umfassend folgende Schritte:

    - Herstellung mindestens einer Spinnlösung durch die gemeinsame Auflösung in einem Lösungsmittel mindestens einer ersten Polymerkomponente und mindestens einer zweiten Polymerkomponente, wobei die erste und die zweite Polymerkomponente unabhängig voneinander ausgewählt ist aus der Gruppe der Polysaccharide und/oder Polysaccharidderivate und wobei die erste Polymerkomponente eine Wasserlöslichkeit, wie im Abschnitt Messmethoden definiert, von mehr als 50 Gew.%, vorzugsweise von mehr als 80 Gew.% und ganz besonders bevorzugt von mehr als 95 Gew.%, aufweist;
    - Zuführen der Spinnlösung zu einer Spinndüse und Extrusion der Spinnlösung durch die Spinndüse in einen Luftspalt zur Erzeugung einer Mehrkomponentenstruktur;
    - Einführen der Mehrkomponentenstruktur in ein Fällbad zur Ausbildung der Mehrkomponentenfaser;
    - Entfernung des Lösungsmittels in mindestens einem Waschbad und
    - Trocknung der Mehrkomponentenfaser.

12. Verfahren nach Anspruch 11, **dadurch gekennzeichnet, dass** keine Vernetzung der ersten Polymerkomponente, insbesondere keine Vernetzung mittels chemischer Vernetzter, durchgeführt wird.

13. Verfahren nach Anspruch 11 oder 12, **dadurch gekennzeichnet, dass** als Lösungsmittel N-Methyl-Morpholin-N-Oxid Monohydrat, und/oder ionische Flüssigkeiten wie beispielsweise EMIMAc, BMIMCl eingesetzt wird.

**Claims**

1. Process for producing a hydrogel-forming multicomponent fibre comprising at least one first fibre component and at least one second fibre component, where the first and second fibre components are selected independently of one another from the group of polysaccharides and/or polysaccharide derivatives, and where the first fibre component is produced from a polysaccharide and/or polysaccharide derivative having a water solubility, as defined in the Test Methods section, of more than 50% by weight, preferably of more than 80% by weight and most preferably of more than 95% by weight, and where the multicomponent fibre has a water solubility, as defined in the Test Methods section, of less than 15% by weight, **characterized in that** the second fibre component is produced from a polysaccharide and/or polysaccharide derivative having a water solubility, as defined in the Test Methods section, of 0% by weight to 5% by weight.

2. Process according to Claim 1, **characterized in that** the hydrogel-forming multicomponent fibre does not have any chemical crosslinking of the first and/or second fibre components via covalent and/or ionic bonds.

3. Process according to Claim 1 or 2, **characterized in that** the hydrogel-forming multicomponent fibre swells by more than 100% in test solution A (according to DIN EN 13726-01, section 3.2.2.3) with increasing fibre diameter (20°C, test solution A, after 10 minutes).

4. Process according to one or more of the preceding claims, **characterized in that** the first and second fibre components are in the form of a mixed phase and preferably form interlooped and/or intermeshed networks with one another.

5. Process according to Claim 4, **characterized in that** the mixed phase and/or the interlooped and/or intermeshed network exist as a component of a core-shell arrangement, or of a segmented arrangement, especially a segmented pie, side-by-side and/or island-in-the-sea arrangement.

6. Process according to one or more of the preceding claims, **characterized in that** the hydrogel-forming multicomponent fibre is obtained by co-dissolution of at least one first and one second polymer component, where the first and second polymer components are selected independently of one another from the group of polysaccharides and/or polysaccharide derivatives, and where the first polymer component is a polysaccharide and/or polysaccharide derivative having a water solubility, as defined in the Test Methods section, of more than 50% by weight.

7. Process according to one or more of the preceding claims, **characterized in that** the hydrogel-forming multicomponent fibre has a fineness-based tear resistance in the conditioned state between 10 cN/tex and 40 cN/tex and/or a fineness-based elongation at break in the conditioned state between 5% and 15% and/or an absorption capacity, measured in accordance with DIN 53923 for test solution A (according to DIN EN 13726-01, section 3.2.2.3) between 5 g/g and 30 g/g.

8. Process according to one or more of the preceding claims, **characterized in that** the ratio of amounts of first and second fibre components is between 80:20 and 10:90.

9. Process according to one or more of the preceding claims, **characterized in that** the first fibre component is selected from the group of cellulose derivatives, for example cellulose ethers, especially carboxymethylcellulose, hydroxypropylcellulose and/or sulfoalkylcellulose, and/or **in that** the second fibre component is selected from the group of polysaccharides and/or polysaccharide derivatives having a water solubility of 0% by weight to 5% by weight.

10. Process according to one or more of the preceding claims, **characterized in that** the first fibre component is selected from the group of cellulose ethers, especially carboxymethylcellulose, hydroxypropylcellulose and/or sulfoalkylcellulose, and/or **in that** the second fibre component is selected from the group of nonionic polysaccharides, especially cellulose.

11. Process according to one or more of the preceding claims, comprising the following steps:

- producing at least one spinning solution by the co-dissolution in a solvent of at least one first polymer component and at least one second polymer component, where the first and second polymer components are selected independently of one another from the group of polysaccharides and/or polysaccharide derivatives and where the first polymer component has a water solubility, as defined in the Test Methods section, of more than 50% by weight, preferably of more than 80% by weight and most preferably of more than 95% by weight;
- feeding the spinning solution to a spinneret and extruding the spinning solution through the spinneret into an air gap to create a multicomponent structure;
- introducing the multicomponent structure into a precipitation bath to form the multicomponent fibre;
- removing the solvent in at least one wash bath and
- drying the multicomponent fibre.

12. Process according to Claim 11, **characterized in that** no crosslinking of the first polymer component, especially no crosslinking by means of chemical crosslinkers, is conducted.

13. Process according to Claim 11 or 12, **characterized in that** the solvent used is N-methylmorpholine N-oxide monohydrate and/or ionic liquids, for example EMIMAc, BMIMCl.

**Revendications**

1. Procédé de fabrication d'une fibre multicomposant formant un hydrogel, comprenant au moins un premier composant de la fibre et au moins un deuxième composant de la fibre, le premier et le deuxième composants de la fibre étant indépendamment l'un de l'autre choisis dans le groupe des polysaccharides et/ou des dérivés polysaccharidiques, et le premier composant de la fibre étant fabriqué à partir d'un polysaccharide et/ou d'un dérivé polysaccharidique ayant une solubilité dans l'eau, telle que définie au paragraphe Méthodes de mesure, supérieure à 50 % en poids, de préférence supérieure à 80 % en poids et idéalement supérieure à 95 % en poids, et la fibre multicomposant présentant une solubilité dans l'eau, telle que définie au paragraphe Méthodes de mesure, inférieure à 15 % en poids, **caractérisé en ce que** le deuxième composant est fabriqué à partir d'un polysaccharide et/ou d'un dérivé polysaccharidique ayant une solubilité dans l'eau, telle que définie au paragraphe Méthodes de mesure, de 0 % en poids à 5 % en poids.

2. Procédé selon la revendication 1, **caractérisé en ce que** la fibre multicomposant formant un hydrogel ne présente aucune réticulation chimique du premier et/ou du deuxième composants par des liaisons covalentes et/ou ioniques.

3. Procédé selon l'une quelconque des revendications 1 ou 2, **caractérisé en ce que** la fibre multicomposant formant un hydrogel gonfle de plus de 100 % dans la solution de mesure A (selon DIN EN 13726-01, paragraphe 3.2.2.3), par grossissement du diamètre de la fibre (20 °C, solution d'essai A, après 10 minutes).

4. Procédé selon l'une ou plusieurs quelconques des revendications précédentes, **caractérisé en ce que** le premier et le deuxième composants de la fibre se présentent sous forme d'une phase mixte et forment de préférence l'un avec l'autre des réseaux d'entrelacement et/ou d'entremêlement.

5. Procédé selon la revendication 4, **caractérisé en ce que** la phase mixte et/ou le réseau d'entrelacement et/ou d'entremêlement se présentent sous forme d'un composant d'une structure cœur-gaine, d'une structure segmentée, en particulier d'une structure en parts de tarte, d'une structure côte à côte et/ou d'une structure en îlots dans la mer.

6. Procédé selon l'une ou plusieurs quelconques des revendications précédentes, **caractérisé en ce que** la fibre multicomposant formant un hydrogel est obtenue par dissolution commune d'au moins un premier et un deuxième composants polymères, le premier et le deuxième composants polymères étant indépendamment de l'autre choisis dans le groupe des polysaccharides et/ou des dérivés polysaccharidiques, et le premier composant polymère étant un polysaccharide et/ou un dérivé polysaccharidique ayant une solubilité dans l'eau, telle que définie au paragraphe Méthodes de mesure, supérieure à 50 % en poids.

7. Procédé selon l'une ou plusieurs quelconques des revendications précédentes, **caractérisé en ce que** la fibre multicomposant formant un hydrogel présente une résistance en traction, rapportée à la finesse, à l'état conditionné entre 10 cN/tex et 40 cN/tex et/ou un allongement à la rupture, rapporté à la finesse, à l'état conditionné entre 5 % et 15 % et/ou un pouvoir d'absorption, mesuré selon DIN 53923 pour la solution d'essai A (selon DIN EN 13726-01, paragraphe 3.2.2.3) entre 5 g/g et 30 g/g.

8. Procédé selon l'une ou plusieurs quelconques des revendications précédentes, **caractérisé en ce que** le rapport entre la quantité du premier et du deuxième composants de la fibre est compris entre 80:20 et 10:90.

9. Procédé selon l'une ou plusieurs quelconques des revendications précédentes, **caractérisé en ce que** le premier composant de la fibre est choisi dans le groupe des dérivés cellulosiques, par exemple les éthers de cellulose, en particulier la carboxyméthylcellulose, l'hydroxypropylcellulose et/ou une sulfoalkylcellulose et/ou **en ce que** le deuxième composant de la fibre est choisi dans le groupe des polysaccharides et/ou des dérivés polysaccharidiques ayant une solubilité dans l'eau de 0 % en poids à 5 % en poids.

10. Procédé selon l'une ou plusieurs quelconques des revendications précédentes, **caractérisé en ce que** le premier composant de la fibre est choisi dans le groupe des éthers de cellulose, en particulier la carboxyméthylcellulose, l'hydroxypropylcellulose et/ou une sulfoalkylcellulose et/ou **en ce que** le deuxième composant de la fibre est choisi dans le groupe des polysaccharides non ioniques, en particulier la cellulose.

11. Procédé selon l'une ou plusieurs quelconques des revendications précédentes, comprenant les étapes suivantes :

- fabrication d'au moins une solution de filage par dissolution commune dans un solvant d'au moins un premier composant polymère et d'au moins un deuxième composant polymère, le premier et le deuxième composants polymères étant indépendamment l'un de l'autre choisis dans le groupe des polysaccharides et/ou des dérivés polysaccharidiques, le premier composant polymère présentant une solubilité dans l'eau, telle que définie au paragraphe Méthodes de mesure, supérieure à 50 % en poids, de préférence supérieure à 80 % en poids et idéalement supérieure à 95 % en poids ;

- amenée de la solution de filage à une filière et extrusion de la solution de filage par la filière dans une fente d'air pour produire une structure multicomposant ;

- introduction de la structure multicomposant dans un bain de coagulation, pour formation de la fibre multicomposant ;

- élimination du solvant dans au moins un bain de lavage et

- séchage de la fibre multicomposant.

12. Procédé selon la revendication 11, **caractérisé en ce qu'**aucune réticulation du premier composant polymère, en particulier aucune réticulation à l'aide d'un réticulant chimique, n'est mise en œuvre.

13. Procédé selon l'une quelconque des revendications 11 ou 12, **caractérisé en ce qu'**on utilise comme solvant le N-méthyl-morpholine-N-oxyde monohydraté, et/ou des liquides ioniques tels par exemple que l'EMIMAc, le BMIMCI.

Figur 1

Figur 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- GB 2518199 A
- US 20080082065 A
- US 20140322512 A1
- US 20050136253 A1
- US 6548730 B
- AU 757461 B
- WO 0001425 A1
- EP 1091770 A1
- US 4199367 A
- US 4289824 A
- US 2005101900 A1
- CN 103060946 A
- US 2016121013 A1

### In der Beschreibung aufgeführte Nicht-Patentliteratur

- **T. RÖDER** ; **R. MÖSLINGER** ; **U. MAIS** ; **B. MORGENSTERN** ; **U. GLATTER**. Charakterisierung der Lösungsstrukturen in technisch relevanten Celluloselösungen. *Lenzinger Berichte*, 2003, vol. 82, 118-127
- **R. MARON** ; **CH. MICHELS** ; **E. TAEGER**. Investigations for preparation of cellulose solutions in NMMO and the following forming. *Lenzinger Berichte*, 1994, vol. 74, 27-29
- **D. COLE** ; **A. JONES**. Solvent-Spun Fibre - A New Member of the Cellulose Fibre Family. *Lenzinger Berichte*, 1990, vol. 69, 73-77